(19) Europäisches Patentamt
European Patent Office
Office européen des brevets

(11) EP 4 144 779 A1

(12) **EUROPEAN PATENT APPLICATION**

(43) Date of publication:
**08.03.2023 Bulletin 2023/10**

(21) Application number: **22192441.8**

(22) Date of filing: **26.08.2022**

(51) International Patent Classification (IPC):
***C08G 18/24*** *(2006.01)* ***C08G 18/38*** *(2006.01)*
***C08G 18/76*** *(2006.01)* ***G02C 7/04*** *(2006.01)*

(52) Cooperative Patent Classification (CPC):
**C08G 18/7642; C08G 18/246; C08G 18/3876**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.09.2021 KR 20210116828**

(71) Applicant: **SKC Co., Ltd.**
**Suwon-si, Gyeonggi-do 16336 (KR)**

(72) Inventors:
- **PAI, Jae Young**
  **16338 Suwon-si (KR)**
- **KIM, Jeong Moo**
  **16338 Suwon-si (KR)**
- **HAN, Hyuk Hee**
  **16338 Suwon-si (KR)**
- **YOU, Kyeong Hwan**
  **16338 Suwon-si (KR)**
- **MYUNG, Jung Hwan**
  **16338 Suwon-si (KR)**
- **CHOI, Eui Jun**
  **16338 Suwon-si (KR)**
- **SHIN, Jung Hwan**
  **16338 Suwon-si (KR)**

(74) Representative: **Stolmár & Partner**
**Patentanwälte PartG mbB**
**Blumenstraße 17**
**80331 München (DE)**

(54) **XYLYLENE DIISOCYANATE COMPOSITION AND OPTICAL POLYMERIZABLE COMPOSITION INCLUDING THE SAME**

(57) The xylylene diisocyanate composition according to exemplary examples includes xylylene diisocyanate (XDI), and an acidity adjusting agent having a boling point of 110 °C or higher, wherein the composition has an acidity of more than 100 ppm and 1,000 ppm or less based on a total weight of xylylene diisocyanate (XDI). A polymerization reaction rate is controlled through regulation of the acidity so that an optical lens having high transmittance and improved optical uniformity may be manufactured.

EP 4 144 779 A1

## Description

[BACKGROUND OF THE INVENTION]

1. Field of the Invention

The present invention relates to a xylylene diisocyanate composition and an optically polymerizable composition including the same, and more specifically, to a xylylene diisocyanate composition prepared through a reaction of an amine salt and an optically polymerizable composition including the same.

2. Description of the Related Art

A diisocyanate compound is widely used, for example, as a raw material for preparation of a polyurethane resin. For example, the diisocyanate compound is used for manufacturing an optical lens in which a polyurethane resin is used, and physical properties of the diisocyanate compound as the manufacturing raw material may directly affect optical properties such as transparency and refractive index of an optical lens.

For example, a polythiourethane resin prepared by reacting a polythiol compound and a diisocyanate compound may be utilized as a base material of the optical lens.

Among the diisocyanate compounds, xylylene diisocyanate (XDI) is widely used in consideration of chemical and optical properties such as reactivity and transparency.

For example, a polymerizable composition for an optical lens may be produced by preparing a composition including XDI and mixing it with a composition including a polythiol compound. In consideration of the stability of XDI and appropriate reactivity with the polythiol compound, it is necessary to design the physical properties of the composition of XDI, a synthesis process thereof and the like.

For example, Korean Published Patent Application No. 2012-0076329 discloses a urethane-based optical material prepared using an isocyanate compound. However, physical properties of the isocyanate composition itself are not under consideration in the above patent.

[SUMMARY OF THE INVENTION]

An object according to the exemplary embodiments is to provide a xylylene diisocyanate composition having improved reaction stability and optical properties, and a method for preparation thereof.

In addition, another object according to exemplary embodiments is to provide an optically polymerizable composition which includes a xylylene diisocyanate composition having improved reaction stability and optical properties.

Further, another object according to exemplary embodiments is to provide an optical product manufactured from the above optically polymerizable composition.

To achieve the above objects, according to an aspect of the present invention, there is provided a xylylene diisocyanate composition, including xylylene diisocyanate (XDI), and an acidity adjusting agent having a boiling point of 110 °C or higher, wherein the composition has an acidity of more than 100 ppm and 1,000 ppm or less based on a total weight of xylylene diisocyanate (XDI).

In exemplary embodiments, a chlorine content of the composition may be less than 100 ppm.

In exemplary embodiments, the chlorine content of the composition may range from 10 ppm to 95 ppm.

In exemplary embodiments, a change in the acidity before and after storage in a dark room at 25 °C for 3 months may be 40 ppm or less.

In exemplary embodiments, a transmittance to a 380 nm wavelength light after storage in a dark room at 25 °C for 3 months may be 99% or more.

In exemplary embodiments, the acidity adjusting agent may include at least one inorganic acid compound selected from the group consisting of halogen acid, sulfuric acid, phosphoric acid and phosphoric acid ester-based compounds.

In exemplary embodiments, the acidity adjusting agent may include at least one organic acid compound selected from the group consisting of acetic acid, benzoic acid, trifluoroacetic acid (TFA), fatty acid, and aromatic carboxylic acid halide.

In exemplary embodiments, the acidity adjusting agent may include at least one solid acid selected from the group consisting of clay, silica alumina, cation exchange resin, acid-attached silica gel, acid-attached alumina, aluminum oxide, and vanadium oxide.

In exemplary embodiments, the acidity adjusting agent may include a cyclic amine compound or a tertiary amine compound.

In exemplary embodiments, the added amount of the acidity adjusting agent may range from 300 ppm to 4,000

ppm.

In addition, according to another aspect of the present invention, there is provided an optically polymerizable composition, including: a xylylene diisocyanate composition which includes xylylene diisocyanate (XDI), and an acidity adjusting agent having a boiling point of 110 °C or higher, wherein the composition has an acidity of more than 100 ppm and 1,000 ppm or less based on a total weight of xylylene diisocyanate (XDI); a polythiol compound; and an additive.

In exemplary embodiments, a chlorine content of the xylylene diisocyanate composition may be less than 100 ppm.

In exemplary embodiments, the additive may include at least one selected from the group consisting of a releasing agent, a reaction catalyst, a thermal stabilizer, an ultraviolet absorber, and a bluing agent.

Further, according to another aspect of the present invention, there is provided a method for preparation of a xylylene diisocyanate composition, including: synthesizing xylylene diisocyanate (XDI) from xylylenediamine to form a preliminary composition containing XDI; and adjusting an acidity of the preliminary composition in a range of more than 100 ppm and 1,000 ppm or less.

In exemplary embodiments, the step of adjusting the acidity of the preliminary composition may include adding an acidic acidity adjusting agent when the acidity of the preliminary composition is 100 ppm or less, and adding a basic acidity adjusting agent when the acidity of the preliminary composition is more than 1,000 ppm.

According to the above-described embodiments, the xylylene diisocyanate composition has an acidity of more than 100 ppm and 1,000 ppm or less, and may provide improved stability and a polymerization reaction rate in an appropriate range with the polythiol-based compound.

Accordingly, an optical lens having a high transmittance and improved optical uniformity with substantially removed clouding and striae phenomena, may be manufactured.

[DETAILED DESCRIPTION OF THE INVENTION]

Hereinafter, embodiments of the present application will be described in detail. However, since the present invention may include various forms with addition of various changes, specific embodiments are illustrated in the drawings and described in detail in the text. However, this is not intended to limit the present invention to the specific forms disclosed above but it should be understood to include all modifications, equivalents and substitutes within the spirit and scope of the present invention.

Unless defined otherwise, all terms used herein, including technical and scientific terms, have the same meaning as commonly understood by one of ordinary skill in the art to which this invention belongs. Terms such as those defined in commonly used dictionaries should be interpreted to have meanings consistent with the context of the related art, and are not to be interpreted in an ideal or excessively formal meaning unless explicitly defined in the present application.

According to one aspect through the present invention, a composition including xylylene diisocyanate (XDI) is provided (hereinafter, may be abbreviated as an XDI composition).

According to exemplary embodiments, the XDI composition may include XDI, and may have an acidity of more than 100 ppm and 1,000 ppm or less based on a total weight of XDI.

The term "acidity" as used herein may refer to an amount of acid to be free by a reaction with alcohol at room temperature, which is a value expressed as a ratio to the total weight of XDI in terms of HC1.

XDI included in the XDI composition may be reacted with a polythiol-based compound such as a trifunctional thiol compound and/or a tetrafunctional thiol compound, thus to obtain a polythiourethane resin. According to exemplary embodiments, the acidity of the XDI composition may be adjusted as a factor affecting the stability of XDI and reactivity with the polythiol-based compound.

For example, when the acidity of the XDI composition is excessively increased, the polymerization reactivity with the polythiol-based compound may be excessively decreased. Thereby, a process yield of the polythiourethane resin for manufacturing an optical lens may be reduced. Further, clouding phenomenon may be caused in a casting process for molding a lens.

When the acidity of the XDI composition is excessively decreased, the polymerization reactivity with the polythiol-based compound may be excessively increased. Thereby, instead of the desired polythiourethane resin, other by-products, for example, in the form of oligomers or polymers may be increased, which in turn may lead to striae of the lens. Further, the self-reactivity of XDI is also increased, which may cause clouding phenomenon during long-term storage. Accordingly, as described below, the transmittance of the composition stock solution may be decreased when stored in a dark room for a long period of time of 3 months at room temperature (25 °C). Further, during long-term storage, the desired target acidity may fluctuate due to its own reaction, such that acidity control cannot be implemented.

In consideration of the above aspects, according to exemplary embodiments, the acidity of the XDI composition may be adjusted to more than 100 ppm and 1,000 ppm or less. Therefore, it is possible to maintain appropriate reactivity with the polythiol-based compound to inhibit cloudiness during lens casting while preventing lens striae. Further, it is possible to inhibit a decrease in the transmittance and a change in the acidity of the XDI composition by securing storage

characteristics of the XDI composition.

In one embodiment, the acidity of the XDI composition may be more than 100 ppm and 800 ppm, preferably, 110 ppm to 800 ppm, and more preferably 110 ppm to 700 ppm or 110 ppm to 500 ppm. For example, the acidity of the XDI composition may range from 110 ppm to 300 ppm, preferably, 110 ppm to 250 ppm, and more preferably, 110 ppm to 200 ppm.

In one embodiment, the acidity of the XDI composition may range from 300 ppm to 700 ppm, or 250 ppm to 500 ppm.

In some embodiments, the change in the acidity of the XDI composition after storage in the dark room at 25 °C for 3 months may be 200 ppm or less. For example, the change in the acidity of the XDI composition after storage in the dark room at 25 °C for 3 months may range from 1 ppm to 200 ppm, 1 ppm to 150 ppm or 1 ppm to 100 ppm.

Preferably, the change in the acidity of the XDI composition after storage in the dark room at 25 °C for 3 months is 40 ppm or less, more preferably, 30 ppm or less, 20 ppm or less, or 15 ppm or less.

For example, the change in the acidity of the XDI composition may range from 1 ppm to 40 ppm, 1 ppm to 35 ppm, or 1 ppm to 20 ppm, preferably, 1 ppm to 15 ppm, and more preferably, 1 ppm to 10 ppm.

The chlorine content may be adjusted along with the acidity of the XDI composition. Lens yellowing may be caused if the chlorine content in the XDI composition is increased. Further, chlorine ions included in the composition may act as an acidity variable factor.

Accordingly, when the chlorine content in the composition is increased, the acidity of the composition adjusted to a predetermined range as described above is changed, and thus the acidity of the desired target range may not be easily implemented. Further, when the composition is stored for a long time, the acidity may be changed due to chlorine, such that desired properties of the lens may not be obtained.

In some embodiments, the chlorine content in the XDI composition may be less than 100 ppm. Preferably, the chlorine content in the XDI composition may be 95 ppm or less.

In one embodiment, the chlorine content in the XDI composition may be maintained at 10 ppm or more and less than 100 ppm. In this case, it is possible to prevent an excessive increase of process load in the distillation and purification process of the XDI composition and to stably maintain the chlorine content in the corresponding range. In one embodiment, the chlorine content in the XDI composition may be maintained in a range of 10 ppm to 95 ppm, 10 to 80 ppm, and preferably 30 ppm to 80 ppm or 40 ppm to 80 ppm.

According to exemplary embodiments, an acidity adjusting agent to finely adjust the acidity within the above-described range may be added to the XDI composition, and a compound having a boiling point of 110 °C or higher may be used as the acidity adjusting agent.

As the acidity adjusting agent having a boiling point of 110 °C or higher is used, a change in the content of the acidity adjusting agent during distillation in the XDI composition manufacturing process may be inhibited. Accordingly, it is possible to stably maintain the acidity and chlorine content in the above-described ranges. Further, using the acidity adjusting agent having a high boiling point can inhibit side reactions caused by the acidity adjusting agent, improve long-term storage of the XDI composition, and maintain a desired acidity range for a long period of time.

In one embodiment, a compound having a boiling point of 110 °C to 500 °C, 110 °C to 400 °C, or 110 °C to 300 °C may be used as the acidity adjusting agent. For example, the boiling point of the acidity adjusting agent may range from 110 °C to 250 °C, or 110 °C to 200 °C.

In some embodiments, the transmittance of the XDI composition to 380 nm wavelength light after 3 months storage in a dark room at 25 °C may be 99% or more.

The acidity adjusting agent may include an inorganic acid compound, an organic acid compound, or a solid acid.

Examples of the inorganic acid compound may include halogen acids such as hydrochloric acid, bromic acid and iodic acid, sulfuric acid, phosphoric acid, and phosphoric acid-based derivatives.

In one embodiment, the phosphoric acid-based derivative may include a phosphoric acid ester-based compound such as a phosphate-based compound or a phosphonate-based compound. For example, the phosphoric acid-based derivative may include a compound of Formula 1 below.

[Formula 1]

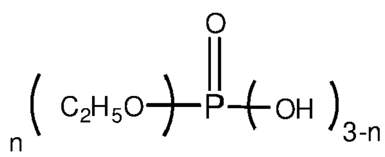

In Formula 1, n is 1 or 2.

Examples of the organic acid compound may include acetic acid, benzoic acid, formic acid, trifluoroacetic acid (TFA), fatty acid, aromatic carboxylic acid halide (e.g., benzoyl halide, phenylacetyl halide, phthaloyl halide, terephthaloyl halide, isop taloyl halide) and the like.

Examples of the solid acid may include acid clay, silica alumina, a cation exchange resin, acid-attached silica gel, or a solid acid such as alumina, aluminum oxide, or vanadium oxide.

In some embodiments, the acidity adjusting agent may include a basic compound which is not substantially reactive with XDI. For example, the acidity adjusting agent may include a cyclic amine such as imidazole, tetrazole, pyridine, and the like, or a tertiary amine such as N,N-dimethylaniline ($PhNMe_2$).

In some embodiments, an added amount or content of the acidity adjusting agent may be regulated in consideration of the above-described acidity range and chlorine content, for example, may range from 300 ppm to 4,000 ppm.

In one embodiment, the added amount or content of the acidity adjusting agent may range from 300 ppm to 2,000 ppm, preferably 300 ppm to 1,000 ppm, and more preferably 300 ppm to 700 ppm or 300 ppm to 500 ppm.

In one embodiment, the added amount or content of the acidity adjusting agent may range from 500 ppm to 2,000 ppm, and preferably 500 ppm to 1,000 ppm. Alternately, the added amount or content of the acidity adjusting agent may range from 700 ppm to 2,000 ppm, and preferably 700 ppm to 1,000 ppm.

In one embodiment, the added amount or content of the acidity adjusting agent may range from 400 ppm to 3,500 ppm, and preferably 500 ppm to 3,000 ppm.

In one embodiment, XDI content in the XDI composition may be 90 wt.% or more, 95 wt.% or more, or 99 wt.% or more, for example, 99 wt.% or more but less than 100 wt.%. In one embodiment, the acidity adjusting agent may be added within a range capable of obtaining acidity in the above-described range.

According to exemplary embodiments, a method for preparation of an XDI composition including the following steps, processes or actions may be provided.

The method for preparation of an XDI composition according to exemplary embodiments may include at least one of the steps, processes or actions described as S10 and S20 below. It should be understood that the following terms "S10" and S20" are used to distinguish the processes for the convenience of description, and are not intended to limit the order of the processes. For example, some or all of the processes of S10 and S20 below may be sequentially performed, and may be performed by changing the order according to process conditions.

S10) Obtaining a preliminary composition containing XDI through a synthesis process of XDI;
S20) Confirming the acidity of the preliminary composition, and if it has an acidity of 100 ppm or less or more than 1,000 ppm, adjusting the acidity to obtain an XDI composition having an acidity of more than 100 ppm and 1,000 ppm or less;

For example, xylylene diisocyanate (XDI) may be synthesized from xylylene diamine in step S10.

In some embodiments, XDI may be synthesized from xylylenediamine by a phosgene method. For example, xylylenediamine may be reacted with concentrated hydrochloric acid in a solvent to form an amine salt. Herein, XDI may be synthesized by reacting the amine salt with phosgene ($COCl_2$) (see Scheme 1 below).

[Scheme 1]

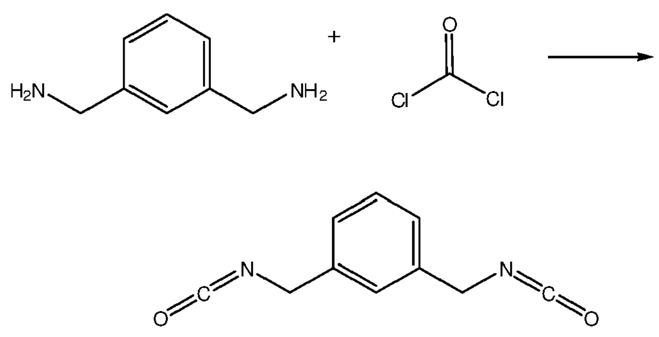

In some embodiments, XDI may be synthesized from xylylenediamine by a biphosgene method. For example, xylylenediamine may be reacted with concentrated hydrochloric acid to form an amine salt. Then, the amine salt may be reacted with halodialkyl carbonate to form biscarbamate. Thermally decomposing or degassing the biscarbamate in the presence of a catalyst may synthesize XDI (see Scheme 2 below).

[Scheme 2]

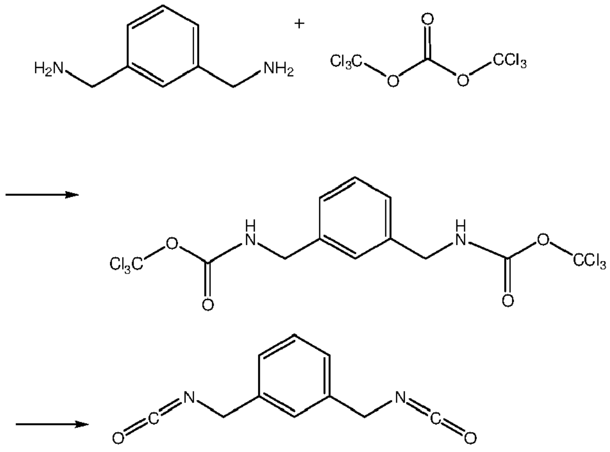

As shown in Scheme 2 above, bis(trichloromethyl)carbonate (BTMC) may be used as an example of the halodialkyl carbonate.

For example, a first solution in which the amine salt is dissolved in an inert solvent may be prepared, and a second solution in which the halodialkyl carbonate is dissolved in an inert solvent may be prepared. The biscarbamate synthesis reaction may be executed while the second solution is added dropwise to the first solution in the reactor. The temperature in the reactor may be maintained, for example, in a range of about 120 °C to 150 °C.

Thereafter, a degassing process may be performed by supplying an inert gas to the reaction solution while maintaining the temperature in the above range. Then, the reaction solution may be cooled, followed by filtration and drying processes, thus to obtain XDI composition.

In some embodiments, a distillation process may be further performed to remove the inert solvent and extract XDI. For example, first distillation for removing the inert solvent and second distillation for extracting XDI may be sequentially performed.

The first distillation temperature may be appropriately adjusted according to the boiling point of the inert solvent. The second distillation temperature may be a temperature of the boiling point or higher of XDI.

In some embodiments, the first distillation temperature may be 100 °C or lower, for example, 50 °C to 90 °C, and preferably 50 °C to 80 °C. The second distillation temperature may be 115 °C or higher, for example, 120 °C to 150 °C, and preferably 120 °C to 140 °C.

The first distillation and the second distillation may be performed under a pressure condition of 1 torr or less, and preferably at a pressure condition of 0.5 torr or less.

The inert solvent may include an organic solvent which is not substantially reactive with the amine salt, XDI and the halodialkyl carbonate. Further, an organic solvent having a lower boiling point than XDI may be used for performing the above-described distillation process.

In one embodiment, the inert solvent may include chlorinated aromatic hydrocarbons such as monochlorobenzene, dichlorobenzene, trichlorobenzene, chloroethylbenzene and the like.

According to exemplary embodiments, the acidity of the XDI-containing preliminary composition prepared as described above in step S20 may be measured and adjusted. For example, when the preliminary composition has an acidity of 100 ppm or less or more than 1,000 ppm, it is adjustable to an acidity of more than 100 ppm and 1,000 ppm or less.

When the acidity of the preliminary composition is 100 ppm or less, an acidity adjusting agent may be added. The acidity adjusting agent may include the above-described inorganic acid compound, organic acid compound, or solid acid.

Preferably, in consideration of fine acidity control, a liquid organic acid compound may be used.

When the acidity of the preliminary composition exceeds 1,000 ppm, the above-described basic compound may be added.

In some embodiments, when the acidity of the preliminary composition is more than 100 ppm and 1,000 ppm or less, the preliminary composition may be used as the XDI composition without addition of the acidity adjusting agent.

According to one aspect through the present invention, there is provided an optically polymerizable composition including the XDI composition prepared as described above.

The optically polymerizable composition may include a polythiol-based compound and the XDI composition.

The polythiol-based compound may include a trifunctional polythiol compound and/or a tetrafunctional polythiol compound.

Non-limiting examples of the trifunctional polythiol compound may include a compound represented by Formula 1 below.

[Formula 1]

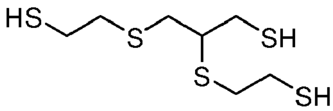

The trifunctional polythiol compound may be synthesized from, for example, a polyol compound obtained through a reaction with 2-mercaptoethanol and epihalohydrin.

After the polyol compound is reacted with thiourea under acidic conditions to produce a thiuronium salt, a trifunctional polythiol compound may be prepared through hydrolysis under basic conditions.

Non-limiting examples of the tetrafunctional polythiol compound may include compounds represented by Formulas 2-1 to 2-3 below.

[Formula 2-1]

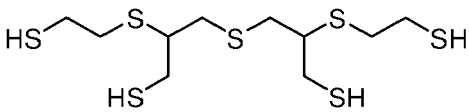

[Formula 2-2]

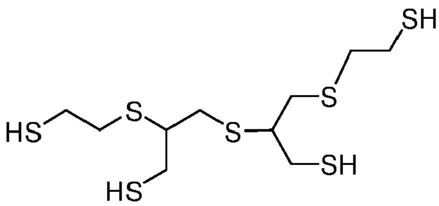

[Formula 2-3]

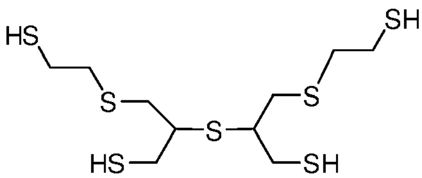

The tetrafunctional polythiol compound may be synthesized from, for example, a polyol compound obtained through a reaction with 2-mercaptoethanol and epihalohydrin. The polyol compound may be reacted with a metal sulfide to produce a tetrafunctional polyol intermediate. After the tetrafunctional polyol intermediate is reacted with thiourea under acid conditions to produce a thiuronium salt, a tetrafunctional polythiol compound may be prepared by hydrolysis under basic conditions.

The optically polymerizable composition may further include additives such as a releasing agent, a reaction catalyst, a thermal stabilizer, an ultraviolet absorber, a bluing agent and the like.

Examples of the releasing agent may include: a fluorine-based nonionic surfactant having a perfluoroalkyl group, a hydroxyalkyl group or a phosphoric acid ester group; a silicone-based nonionic surfactant having a dimethylpolysiloxane group, a hydroxyalkyl group or a phosphoric acid ester group; alkyl quaternary ammonium salts such as trimethylcetyl ammonium salt, trimethylstearyl, dimethylethylcetyl ammonium salt, triethyldodecyl ammonium salt, trioctylmethyl ammonium salt and diethylcyclohexadodecyl ammonium salt; acidic phosphoric acid ester or the like. These may be used alone or in combination of two or more thereof.

As the reaction catalyst, the catalysts used in the polymerization reaction of the polythiourethane resin may be used. For example, dialkyltin halide-based catalysts, such as dibutyltin dichloride and dimethyltin dichloride; dialkyltin

dicarboxylate-based catalysts such as dimethyltin diacetate, dibutyltin dioctanoate, and dibutyltin dilaurate; dialkyltin dialkoxide-based catalysts such as dibutyltin dibutoxide and dioctyltin dibutoxide; dialkyltin dithioalkoxide-based catalysts such as dibutyltin di(thiobutoxide); dialkyltin oxide-based catalysts such as di(2-ethylhexyl)tin oxide, dioctyltin oxide, and bis(butoxydibutyltin)oxide; dialkyltin sulfide-based catalysts, or the like may be used. These may be used alone or in combination of two or more thereof.

As examples of the ultraviolet absorber, benzophenone, benzotriazole, salicylate, cyanoacrylate, and oxanilide-based compounds, etc. may be used. As examples of the thermal stabilizer, metal fatty acid salt, phosphorus, lead, and organotin-based compounds, etc. may be used. These may be used alone or in combination of two or more thereof.

The bluing agent may be included as a color adjusting agent of the optical material prepared from the polythiourethane resin. For example, the bluing agent may have an absorption band in a wavelength range from orange to yellow in a visible light region.

Examples of the bluing agent may include a dye, a fluorescent whitening agent, a fluorescent pigment, an inorganic pigment, or the like, and may be appropriately selected according to physical properties or resin color required for the optical product to be manufactured. When the dye is used as the bluing agent, for example, a dye having a maximum absorption wavelength of 520 nm to 600 nm, and preferably 540 nm to 580 nm may be used. Preferably, anthraquinone-based dyes are used.

In some embodiments, the polythiol-based compound, the isocyanate-based compound, and the above-described additive may be included in amounts of 40 wt.% to 60% by weight ("wt.%"), 40 wt.% to 60 wt.%, and 0.01 wt.% to 1 wt.%, respectively, based on a total weight of the optically polymerizable composition.

A polythiourethane resin may be produced through a polymerization reaction of the polythiol-based compound and XDI included in the optically polymerizable composition.

As described above, the acidity of the XDI composition used in the optically polymerizable composition is regulated to be more than 100 ppm and 1,000 ppm or less, such that the reactivity or reaction rate with the polythiol-based compound may be appropriately controlled. Accordingly, while suppressing the clouding phenomenon derived from the XDI composition itself, the clouding phenomenon in the optical lens prepared from the optically polymerizable composition can be prevented together.

In some embodiments, the reaction rate of the optically polymerizable composition included in Equation 1 to be described below may be maintained in a range of 0.17 to 0.30. In a preferred embodiment, the reaction rate may be maintained in a range of 0.17 to 0.25, and preferably 0.19 to 0.21.

Further, an optical lens having a uniform refractive index without striae phenomenon may be manufactured through a stable polymerization reaction.

According to one aspect of the present invention, there may be provided an optical product manufactured using the above-described optically polymerizable composition.

For example, after degassing the optically polymerizable composition under reduced pressure, it may be injected into a mold for forming an optical material. Mold injection may be performed, for example, in a temperature range of 10 °C to 40 °C, and preferably 10 °C to 30 °C.

After the mold injection, the temperature may be gradually increased to execute a polymerization reaction of the polythiourethane resin. The polymerization temperature may range from 20 °C to 150 °C, and preferably 25 °C to 125 °C. For example, the maximum polymerization temperature may range from 100 °C to 150 °C, preferably 110 °C to 140 °C, and more preferably 115 °C to 130 °C.

The polymerization time may range from 1 to 10 hours, and preferably 1 to 5 hours.

For example, a lens having uniform optical properties and mechanical properties may be easily obtained by appropriately controlling the reaction rate within the above temperature range.

After completing polymerization, the polymerized polythiourethane resin may be separated from the mold to obtain an optical product. The optical product may be manufactured in the form of a spectacle lens, a camera lens, a light emitting diode, etc. according to a mold shape.

In one embodiment, after separation from the mold, a curing process may be further performed. The curing process may be carried out at a temperature in a range of 100 °C to 150°C, preferably 110 °C to 140 °C, more preferably 115 °C to 130 °C for 1 to 10 hours, and preferably 1 to 3 hours.

The refractive index of the optical product may be adjusted according to the type and/or content ratio of the polythiol-based compound and the isocyanate-based compound used in the optically polymerizable composition. For example, the refractive index of the optical product may be regulated in a range of 1.56 to 1.78, 1.58 to 1.76, 1.60 to 1.78, or 1.60 to 1.76, and preferably in a range of 1.65 to 1.75, or 1.69 to 1.75.

The yellow index (Y.I.) of the optical product according to Equation 2 to be described below may be less than 30, preferably 28 or less, and more preferably 22 or less, or 21 or less.

The optical product may be improved by additionally performing surface treatment such as anti-fouling, color imparting, hard coating, surface polishing, hardness strengthening and the like.

Hereinafter, embodiments provided in the present application will be further described with reference to specific

experimental examples. Examples and comparative examples included in the experimental examples are merely illustrative of the present invention and do not limit the appended claims. Further, it will be apparent to those skilled in the art that various changes and modifications to the examples are possible within the scope and technical spirit of the present invention. Further, it is of course understood that such variations and modifications fall within the scope of the appended claims.

## Preparative Example

### (1) Preparation of xylylenediamine (XDA) hydrochloride salt

1009.4 g (9.46 mol) of a hydrochloric acid 35% solution was introduced into a reactor, and the temperature inside the reactor was cooled to a range of 15 °C to 20 °C while stirring. Then, 600.0 g (4.4 mol) of meta-xylylenediamine (m-XDA) was slowly introduced while maintaining the reactor temperature in a range of 20 °C to 60 °C.

After completing the introduction of m-XDA, the temperature inside the reactor was cooled to a range of 10 °C to 20 °C, and after stirring for 1 hour, 1320.0 g of tetrahydrofuran was added. The temperature inside the reactor was cooled again to a range of -5 °C to 0 °C, and the reaction was executed while additionally stirring for 1 hour.

After completing the reaction, vacuum filtration was performed, followed by drying at a reactor external temperature of 90 °C to 100 °C in a vacuum pump under 0.1 torr condition to remove residual solvent and moisture, thereby obtaining m-XDA hydrochloride.

### (2) Preparation of xylylene diisocyanate composition

800 g of m-XDA hydrochloride prepared in (1) above and 3,550 g of ortho-diclobenzene (ODCB) were added to the reactor, and the internal temperature of the reactor was increased to about 125° C by heating while stirring.

950 g of bis(trichloromethyl) carbonate (BTMC) and 800 g of ODCB were dissolved under stirring at about 60 °C, and then was dropped over 24 hours into reactor which was adjusted to 125 °C to prevent precipitation. Pre-mixing was performed for 4 hours after the end of the dropping.

After completing the reaction, $N_2$ gas was supplied to the reaction solution at a temperature of 125 °C, followed by bubbling, and a degassing process was performed. After the degassing, the reaction solution was cooled to 10 °C and the remaining solid was filtered using Celite 545.

The filtered organic solvent and the synthesized crude XDI were purified by distillation under the following conditions.

1) Removal of organic solvent (ODCB) (first distillation)

- Vacuum 0.5 torr or less
- Distillation column bottom temperature: 60 °C
- Distillation time: 8 hours

2) XDI distillation (second distillation)

- Vacuum 0.5 torr or less
- Distillation column bottom temperature: 120 °C
- Distillation time: 10 hours

XDI compositions according to the examples and the comparative examples were prepared by adding an acidity control agent to the XDI prepared as described above to measure the acidity as described in Table 1 below. Specifically, the acidity of the prepared preliminary composition containing XDI was first measured, and an acidity adjusting agent was added while measuring the acidity so that the target acidity described in Table 1 was obtained, thereby preparing an XDI composition.

In Comparative Examples 2, 3 and 8, the acidity and chlorine content were changed by altering the second distillation temperature. In Comparative Examples 2 and 8, the second distillation temperature was altered to 180 °C and 170 °C, respectively, and the second distillation temperature of Comparative Example 3 was regulated to 120 °C.

## Acidity measurement method

20 g of the prepared XDI composition sample was quantified and introduced into a 200 ml beaker, and 100 ml of a solvent (acetone and ethanol mixed in a 1:1 weight ratio) was added thereto, then heated on a hot plate to dissolve

the sample, followed by mixing them for 10 to 20 minutes at room temperature.

Then, using an automatic measuring device (Hiranuma COM-500) and in accordance with JIS K4101, 0.1 mol/L methanolic potassium hydroxide adjusted and expressed using methanol was diluted 10 times to prepare a solution (N/100 methanolic potassium hydroxide solution). Then, acidity was calculated by the following equation with a rising point of a titration curve prepared using the prepared solution as the endpoint.

$$\text{Acidity} = 0.0365 \times (A - B) \times f/S$$

Wherein, A denotes an amount (ml) of N/100 methanolic potassium hydroxide solution required for titration of the sample,

B denotes an amount (ml) of N/100 methanolic potassium hydroxide solution required for blank test,
f denotes a factor of N/100 methanolic potassium hydroxide solution, and
S denotes a weight (g) of the sample.

**2) Preparation of optically polymerizable composition and lens**

49.3 parts by weight ("wt. parts") of 4,8-bis(mercaptomethyl)-3,6,9-trithiaundecane-1,11-dithiol as a polythiol compound, 50.7 wt. parts of xylylene diisocyanate synthesized according to the above preparative example, 0.01 wt. parts of dibutyltin chloride, and 0.1 wt. parts of ZELEC® UN Stepan's phosphoric acid ester releasing agent were uniformly mixed, followed by executing a defoaming process for 1 hour at 600 Pa, to prepare an optically polymerizable composition.

The resin composition filtered through a 3 μm Teflon filter was injected into a mold including a glass mold and a tape. After maintaining the mold at 10 °C to 25 °C for 8 hours, the temperature was slowly increased to 130 °C for 8 hours at a constant rate, and polymerization was executed at 130 °C for 2 hours. After the polymerization was completed, the mold was separated, followed by further curing the product at 120 °C for 2 hours to prepare a lens sample.

**Experimental Example**

**(1) Measurement of chlorine content**

The chlorine content in the XDI compositions of the above examples and comparative examples was measured using a sample combustion apparatus (Analytech/AQF-2100H, Mitsubishi Chemical) and ion chromatography (881 Compact IC Pro, metrohm Co.).

**(2) Determination of change in the acidity**

After storing the XDI compositions of the above examples and comparative examples in a dark room at 25 °C for 3 months, acidity was measured by the above-described method. An extent of change in the acidity was calculated using the measured acidity values before and after storage in the dark room.

**(3) Assessment of cloudiness of XDI composition**

After storing the XDI compositions of the above examples and comparative examples in the dark room at 25 °C for 3 months, a sample was placed in a 10 mm quartz cell, followed by measuring transmittance at 380 nm wavelength and 25 °C. (Transmittance measurement equipment: Lambda 365, Perkinelmer Co.)

**(4) Assessment of physical properties of polymerizable composition/lens**

1) Evaluation of striae

As described above, a lens sample having a diameter of 75 mm, - 8.00D was prepared using the polymerizable composition according to the above examples and comparative examples, and light from a mercury lamp light source was transmitted through the prepared lens sample, followed by projecting the transmitted light on a white plate such that the presence or absence of striae was determined by the presence or absence of contrast. Standards for evaluation are as follows.

○: Striae not observed

△: Partial striae finely observed
x: Clear striae were visually observed

2) Assessment of lens cloudiness

The lens samples of the above examples and comparative examples prepared as described above were irradiated with a projector in a dark room, and the presence or absence of haze and whether to observe opaque materials by the lens were visually confirmed.
Standards for evaluation are as follows.

○: No haze
△: Partial haze observed
x: Clear haze was visually observed as a whole

3) Measurement of polymerization reaction rate (reactivity slope)

Using a non-contact viscometer of EMS-1000 (KEM), the standard viscosity (standard cps) was first confirmed with a viscosity standard solution (Brookfield, 1000 cps, 25 °C). Thereafter, the viscosity was measured at 10 °C for 24 hours for the polymerizable compositions according to the above examples and comparative examples. Using the measured values, Y-axis was defined to be logarithmic in order to form Equation 1 below, wherein X-axis is time and the Y-axis is viscosity, followed by deducing the reaction rate.

[Equation 1]

$$Y = a \times \exp(b \times X)$$

In Equation 1, "a" value represents an initial viscosity (cps), and "b" value represents the reaction rate, and the calculated value was expressed by rounding to the third decimal place of the measured value.

4) Measurement of yellow index (Y.I.)

For the lens samples of the above examples and comparative examples, Y.I was measured using UV/VIS Spectroscopy (PerkinElmer, Model UV/VIS Lambda 365). Specifically, the chromaticity coordinates x and y were measured by transmitting light in a height direction of the plastic circumference (r (radius) x H (height) = 16 mm x 45 mm). Y.I was calculated by Equation 2 below based on the measured values of x and y.

[Equation 2]

$$Y.I = (234 \times x + 106 \times y + 106)/y$$

The measurement results and evaluation results are shown together in Tables 1 and 2 below.

[TABLE 1]

| | Acidity of XDI composition (ppm) | Types of acidity adjusting agent | Added amount of acidity adjusting agent | Boiling point of acidity adjusting agent(°C) | Chlorine content (ppm) |
|---|---|---|---|---|---|
| Example 1 | 115 | E | 1000 | 249 | 45 |
| Example 2 | 228 | E | 2000 | 198 | 51 |
| Example 3 | 133 | G | 500 | 158 | 67 |
| Example 4 | 296 | G | 1000 | 158 | 58 |
| Example 5 | 120 | H | 1000 | 118 | 55 |
| Example 6 | 243 | H | 2000 | 118 | 62 |

(continued)

| | Acidity of XDI composition (ppm) | Types of acidity adjusting agent | Added amount of acidity adjusting agent | Boiling point of acidity adjusting agent(°C) | Chlorine content (ppm) |
|---|---|---|---|---|---|
| Example 7 | 234 | I | 1000 | 203 | 78 |
| Example 8 | 780 | I | 3000 | 203 | 76 |
| Example 9 | 125 | D | 500 | 225 | 125 |
| Example 10 | 222 | D | 1000 | 225 | 241 |
| Example 11 | 185 | C | 1000 | 198 | 286 |
| Comparative Example 1 | 29 | C | 200 | 198 | 86 |
| Comparative Example 2 | 1025 | - | - | - | 1366 |
| Comparative Example 3 | 72 | - | - | - | 90 |
| Comparative Example 4 | 527 | A | 1000 | 69 | 135 |
| Comparative Example 5 | 279 | B | 1000 | 60 | 223 |
| Comparative Example 6 | 108 | F | 1000 | 101 | 43 |
| Comparative Example 7 | 225 | F | 2000 | 101 | 42 |
| Comparative Example 8 | 942 | - | - | - | 1215 |

Specific compounds of the acidity adjusting agents used in Table 1 are as follows.

A: Sulfuryl chloride

B: Trimethylsilyl chloride

C: Benzoyl chloride

D: Phenylacetyl chloride

E: Benzoic acid

F: Formic acid

G: Phophoric acid

H: Acetic acid

I: Ethyl acid phosphate

[TABLE 2]

| | Acidity after storage at 25 °C for 3 months (ppm) | Change in acidity (ppm) | Transmittanc e after storage at 25 °C for 3 months (%) | Physical properties of lens | | | |
|---|---|---|---|---|---|---|---|
| | | | | Striae | Cloudin ess | Reacti on rate (b) | Y.I. |
| Example 1 | 114 | 1 | >99 | ○ | ○ | 0.21 | 20 |
| Example 2 | 215 | 13 | >99 | ○ | ○ | 0.20 | 20 |
| Example 3 | 125 | 8 | >99 | ○ | ○ | 0.21 | 21 |
| Example 4 | 289 | 7 | >99 | ○ | ○ | 0.19 | 21 |
| Example 5 | 102 | 18 | >99 | ○ | ○ | 0.21 | 21 |
| Example 6 | 211 | 32 | >99 | ○ | ○ | 0.20 | 21 |
| Example 7 | 225 | 9 | >99 | ○ | ○ | 0.20 | 20 |
| Example 8 | 768 | 12 | >99 | ○ | ○ | 0.17 | 22 |
| Example 9 | 110 | 15 | >99 | ○ | ○ | 0.21 | 26 |
| Example 10 | 202 | 20 | >99 | ○ | ○ | 0.21 | 28 |
| Example 11 | 178 | 7 | >99 | ○ | ○ | 0.21 | 26 |
| Comparative Example 1 | 28 | 1 | 57 | × | ○ | 0.24 | 23 |
| Comparative Example 2 | 953 | 72 | >99 | ○ | × | 0.14 | 28 |
| Comparative Example 3 | 66 | 6 | 68 | × | ○ | 0.26 | 20 |
| Comparative Example 4 | 338 | 189 | >99 | ○ | ○ | 0.18 | 30 |
| Comparative Example 5 | 125 | 154 | >99 | ○ | ○ | 0.19 | 30 |
| Comparative Example 6 | 45 | 63 | 77 | △ | ○ | 0.23 | 20 |
| Comparative Example 7 | 68 | 157 | 88 | × | ○ | 0.24 | 20 |
| Comparative Example 8 | 899 | 43 | >99 | ○ | △ | 0.16 | 30 |

Referring to Tables 1 and 2, cloudiness in the composition and lens state in the examples having the acidity of more than 100 ppm and 1000 ppm or less was prevented, an appropriate polymerization reaction rate was obtained, and lens striae were suppressed. Further, when the compound having a boiling point of 110 °C or higher was added as the acidity adjusting agent and the composition was stored for a long period of time, the change in the acidity was inhibited while improving the composition transmittance.

Referring to Examples 1 to 8, the chlorine content in the composition was decreased to less than 100 ppm such that lens yellowing was reduced. Further, striae caused by an increase in the reaction rate were more effectively suppressed.

## Claims

1. A xylylene diisocyanate composition, comprising xylylene diisocyanate (XDI), and an acidity adjusting agent having a boiling point of 110 °C or higher,
   wherein the xylylene diisocyanate composition has an acidity of more than 100 ppm and 1,000 ppm or less based

on a total weight of xylylene diisocyanate (XDI).

**2.** The xylylene diisocyanate composition according to claim 1, wherein a chlorine content of the xylylene diisocyanate composition is less than 100 ppm.

**3.** The xylylene diisocyanate composition according to claim 2, wherein the chlorine content of the xylylene diisocyanate composition ranges from 10 ppm to 95 ppm.

**4.** The xylylene diisocyanate composition according to claim 1, wherein a change in the acidity before and after storage in a dark room at 25 °C for 3 months is 40 ppm or less.

**5.** The xylylene diisocyanate composition according to claim 1, wherein a transmittance to a 380 nm wavelength light after storage in a dark room at 25 °C for 3 months is 99% or more.

**6.** The xylylene diisocyanate composition according to claim 1, wherein the acidity adjusting agent comprises at least one inorganic acid compound selected from the group consisting of halogen acid, sulfuric acid, phosphoric acid and phosphoric acid ester-based compounds.

**7.** The xylylene diisocyanate composition according to claim 1, wherein the acidity adjusting agent comprises at least one organic acid compound selected from the group consisting of acetic acid, benzoic acid, trifluoroacetic acid (TFA), fatty acid, and aromatic carboxylic acid halide.

**8.** The xylylene diisocyanate composition according to claim 1, wherein the acidity adjusting agent comprises at least one solid acid selected from the group consisting of clay, silica alumina, cation exchange resin, acid-attached silica gel, acid-attached alumina, aluminum oxide, and vanadium oxide.

**9.** The xylylene diisocyanate composition according to claim 1, wherein the acidity adjusting agent comprises a cyclic amine compound or a tertiary amine compound.

**10.** The xylylene diisocyanate composition according to claim 1, wherein the added amount of the acidity adjusting agent ranges from 300 ppm to 4,000 ppm.

**11.** An optically polymerizable composition, comprising:

a xylylene diisocyanate composition which includes xylylene diisocyanate (XDI), and an acidity adjusting agent having a boiling point of 110 °C or higher, wherein the xylylene diisocyanate composition has an acidity of more than 100 ppm and 1,000 ppm or less based on a total weight of xylylene diisocyanate (XDI);
a polythiol compound; and
an additive.

**12.** The optically polymerizable composition according to claim 11, wherein a chlorine content of the xylylene diisocyanate composition is less than 100 ppm.

**13.** The optically polymerizable composition according to claim 11, wherein the additive includes at least one selected from the group consisting of a releasing agent, a reaction catalyst, a thermal stabilizer, an ultraviolet absorber, and a bluing agent.

**14.** A method for preparation of a xylylene diisocyanate composition, comprising:

synthesizing xylylene diisocyanate (XDI) from xylylenediamine to form a preliminary composition containing XDI; and
adjusting an acidity of the preliminary composition in a range of more than 100 ppm and 1,000 ppm or less.

**15.** The method for preparation of a xylylene diisocyanate composition according to claim 14, wherein the adjusting the acidity of the preliminary composition comprises adding an acidic acidity adjusting agent when the acidity of the preliminary composition is 100 ppm or less, and adding a basic acidity adjusting agent when the acidity of the preliminary composition is more than 1,000 ppm.

Europäisches Patentamt
European Patent Office
Office européen des brevets

# EUROPEAN SEARCH REPORT

Application Number
EP 22 19 2441

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2006 273717 A (MITSUI CHEMICALS INC) 12 October 2006 (2006-10-12) * paragraph [0045]; example 5 * ----- | 1-15 | INV. C08G18/24 C08G18/38 C08G18/76 G02C7/04 |
| X | JP 2019 059823 A (MITSUI CHEMICALS INC) 18 April 2019 (2019-04-18) * paragraph [0150]; table 1 * ----- | 1,6 | |

TECHNICAL FIELDS SEARCHED (IPC)
C08G
G02C

The present search report has been drawn up for all claims

1

| Place of search | Date of completion of the search | Examiner |
|---|---|---|
| Munich | 5 January 2023 | Bergmeier, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 22 19 2441

This annex lists the patent family members relating to the patent documents cited in the above-mentioned European search report. The members are as contained in the European Patent Office EDP file on The European Patent Office is in no way liable for these particulars which are merely given for the purpose of information.

05-01-2023

| Patent document cited in search report | Publication date | Patent family member(s) | Publication date |
| --- | --- | --- | --- |
| JP 2006273717 A | 12-10-2006 | JP 4606918 B2 | 05-01-2011 |
| | | JP 2006273717 A | 12-10-2006 |
| JP 2019059823 A | 18-04-2019 | JP 7025162 B2 | 24-02-2022 |
| | | JP 2019059823 A | 18-04-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20120076329